Europäisches Patentamt

European Patent Office

Office européen des brevets

(11) Publication number: **0 448 095 A1**

# EUROPEAN PATENT APPLICATION

(21) Application number: 91104433.7

(22) Date of filing: 21.03.91

(51) Int. Cl.⁵: **A61K 39/21**, C12N 15/49, C12N 5/10

(30) Priority: 21.03.90 EP 90105313

(43) Date of publication of application:
**25.09.91 Bulletin 91/39**

(84) Designated Contracting States:
**AT BE CH DE DK ES FR GB GR IT LI LU NL SE**

(71) Applicant: **Wolf, Hans Joachim, Prof. Dr.**
**Josef Jägerhuberstrasse 9**
**W-8130 Starnberg(DE)**

(72) Inventor: **Wolf, Hans, Prof. Dr.**
**Josef Jägerhuberstrasse 9**
**W-8130 Starnberg(DE)**
Inventor: **V. Brunn, Albrecht, Dr.**
**Baseler Strasse 70**
**W-8000 München 71(DE)**
Inventor: **Wagner, Ralf, Dipl.-Biol.**
**Steinmetzstrasse 11**
**W-8000 München 71(DE)**
Inventor: **Modrow, Susanne, Dr.**
**Minorstrasse 20 a**
**W-8000 München 71(DE)**

(74) Representative: **Vossius & Partner**
**Siebertstrasse 4 P.O. Box 86 07 67**
**W-8000 München 86(DE)**

(54) **Subregion of the retroviral ENV protein, DNA sequences encoding it and compositions for the diagnosis, prevention or therapy of retrovirus infections.**

(57) In order to reduce the opportunities of HIV to evade vaccine induced immunity, the developement of subunit vaccines must concentrate on those epitopes which are major targets of the immune system. The most dominant neutralization site on the gp120 resides within the third variable domain V3 of the molecule. This epitope was first predicted from our group as the only hypervariable antigenic region without consensus sequence for N-glycosylation and discussed according to its immunological importance. To overcome highly typspecific V3 mediated neutralization described by other laboratories we designed, based on a comparison of V3 sequences derived from different independent virus isolates, a 36 amino acid gp120/V3 consensus peptide. This peptide was tested for its immunological reactivity using independent sera of HIV positive individuals and typespecific anti-V3-antisera. After conversion into DNA this sequence will be an important component of newly designed subunit vaccines.

The technical problem underlying the present invention is to provide compounds corresponding to antigens naturally eliciting neutralizing antibodies for diagnosis, prophylaxis and therapy of retrovirus infections. This invention particularly relates to a newly designed consensus sequence being biologically equivalent to the highly variable and major neutralizing epitope V3 of the HIV-1 envelope protein. This consensus is based on a comparison considering the region of different naturally occuring virus strains and parameters known to be necessary for the 3D configuration of the V3-loop. A 36 amino acids synthetic peptide representing the modelled V3 consensus sequence containing a strictly conserved core-sequence, a T-cell epitope, an ADCC epitope and two highly conserved Cys residues is recognized by at least 85% of randomly selected sera of individuals infected with the retrovirus from which it is derived. The V3 consensus corresponding DNA sequence will be an important component of newly designed subunit vaccines.

To develop diagnostic reagents, therapeutic compounds and vaccines, detailled knowledge about the epitopes reactive with the immune system and about the structures, which are involved in viral infectivity is necessary. Vaccine induced immunity must minimize the opportunities for the virus to escape. For this reason, the development of a subunit vaccine concentrates on the antigenic domains that are main targets for the immune response and which are shared by a wide range of different virus isolates. To include several naturally ocurring variants of the same antigenic epitope, the variability of such domains has to be considered. Several domains have been shown to induce virus neutralizing antibodies in experimental animals when applied in the appropriate form. Amongst them, the most dominant neutralization site resides in the third variable domain of the HIV envelope protein gp120.

## GPGR-V3-loop of gp120

The hypervariable V3-loop of the gp120 was first described from our group as the only hypervariable antigenic region without consensus sequence for N-glycosylation and was discussed in its immunological importance (Modrow et al. 1987). Actually, this region was identified by several laboratories to be the major HIV-1 neutralizing epitope (Palker et al., 1988, Goudsmit et al., 1988, Modrow et al., 1989). It could be correlated with a 24 aa sequence (aa 302-326). A 6 amino acid peptide is sufficient to induce neutralizing antibodies. This sequence contains a 4 aa beta turn motiv, GPGR, that is highly conserved in various isolates (Javaherian et al., 1989). The flanking amino acids seem to be critical for antibody binding and type specific virus neutralization.

Sequence analysis of virus isolates circulating in Europe and USA demonstrated, that V3 heterogeneity is limited (Putney et al., 1989; Goudsmit et al., in press). The majority of the sequenced virus isolates could be classified as variants similar to MN- or SC- like strains. Sequence alignment and subsequent clustering revealed five arbitrary virus families: one consisting of MN, SF, SC, one of CDC4 and NY5, one of RF, one of ELI, Z6 and MAL and one of Z3 (Myers et al., 1989). Based on serum cross reactivities, Goudsmit et al. (1989a) discriminated three virus families: SF and RF; ELI, Z6 and MAL; Z3, CDC4 and NY5.

Geographic analysis of V3 heterogeneity implicates 3 conclusions:
1. MN and SC-type isolates are most common in USA and Europe and belong to different family clusters.
2. MAL and NY5 are clustered in one family and are ubiquitous in USA and Africa.
3. The most different african isolates MAL, Z3 and Z6 indicate much higher linear V3 heterogeneity in Africa than in Europe and USA.

Recently published in vitro and in vivo data (Blattner et al., 1989; Kong et al., 1989) suggested that conformational changes are involved in V3 heterogeneity. Conformational V3 heterogeneity means that virus isolates with conservative or no amino acid exchanges in the V3 epitope aquire resistence to neutralization by strain specific anti V3 antibodies.

A 14 aa sequence (315-329) was characterized as an ADCC target region (Lyerly et al., 1987, 1988). Moreover, it could be shown, that MHC class I restricted cytotoxic T-cells recognize an 18 aa peptide of the V3 region in the mouse model (Takahashi et al., 1988).

How anti-V3-neutralizing antibodies actually inhibit infection and syncytia formation in vitro is not clear to date. Possible hints are given by recent results indicating that processing of gp120 in 2 polypeptides (50 KD, 70 KD) occurs under certain assay conditions . The cleavage site was mapped directly to the conserved sequence GPGRA of the V3-loop. The processed gp120 is still able to bind to the CD4 molecule, but cannot be any longer recognized by anti-V3-neutralizing antibodies. Trypstatin, a Kunitz-type protease inhibitor with a high sequence homology to the gp120/V3 region, inhibits the described protease activity and the following syncytia formation. The V3 dependent processing event, which occurs after association of gp120 with the CD4 molecule seems to be a predisposition for the fusion of the outer viral membrane or the membrane of HIV-1 infected cells with a CD4 target cell. This essential process may explain the inhibitory effect of anti-V3-neutralising antibodies (Hattori et al., 1989). Recent evidence indicates, that the deletion of this loop from an infectious virus clone abolishes its infectivity.

**Disadvantages of using naturally occurring V3 sequences as antigen or immunogen**

Although the importance of the V3-region as a diagnostic antigen or as an immunogen is clearly visible from the background art, the main disadvantage is the variability associated with that region and as a consequence the typespecific immune response. The knowledge about V3 heterogeneity, about geographic distribution of V3-family-clusters and about different epitopes involved in ADCC, CTL and virus neutralization enables us to design V3 consensus sequences with a broader range of antigenicity and immunogenicity than naturally occurring HIV-1 V3 variants. A designed consensus peptide was immunologically evaluated. After translation of the peptide derived sequence into DNA a component of a potential subunit vaccine can be designed. The respective DNA sequence can be included as a compound of potential subunit vaccine.

Thus, the technical problem underlying the present invention is to provide compounds corresponding to antigens naturally eliciting neutralizing antibodies for diagnosis, prophylaxis and therapy of retrovirus infections.

The solution of this technical problem is achieved by providing the embodiments characterized in the claims.

Accordingly the present invention relates to a subregion of the retroviral envelope protein displaying the following features:

a) it is biologically equivalent to the V3 region of the HIV-1 env protein;

b) it is recognized by at least 70% of randomly selected sera of individuals infected with the retrovirus from which it is derived;

c) it contains a strictly conserved oligopeptide;

d) it contains a T-cell epitope;

e) it contains two Cys residues which are essential for the formation of its secondary and /or tertiary structure; and

f) its sequence is designed by using the V3 region of a given HIV-1 env protein and introducing deletions observed in the majority of other HIV-1 isolates, or by comparing the V3 regions of given HIV-1 env proteins and determining a consensus sequence which optionally includes deletions observed in the majority of HIV-1 isolates.

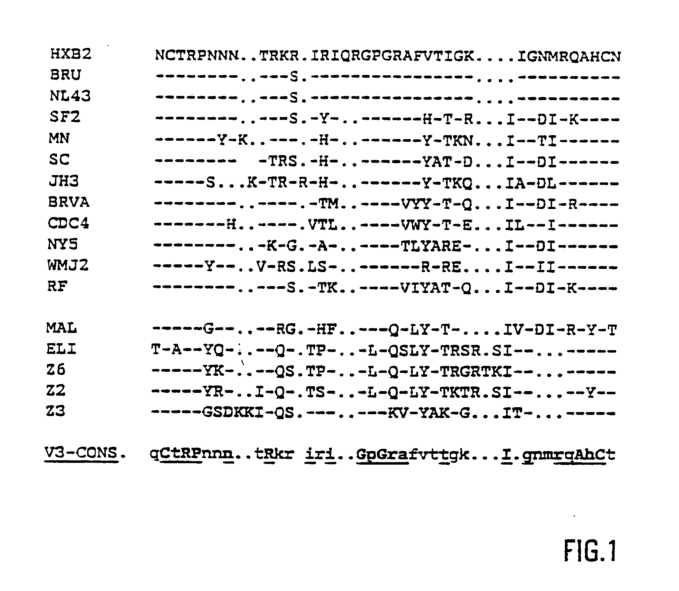

In a preferred embodiment an additional deletion is inserted into the above subregion which corresponds to position 36 of figure 1.

A preferred embodiment of the invention is the V3 region of the major envelope protein gp120 of HIV-1. The above subregion consists of an amino acid sequence corresponding to the amino acid sequence between positions aa294 and aa332 of said gp120 of HIV-1 BH10 isolate.

The above subregion contains a strictly conserved oligopeptide which has a length of about 3 to 10 amino acids.

In a particular preferred embodiment the above subregion contains a strictly conserved oligonucleotide which has the sequence GPGR.

In a further embodiment the above subregion contains the two Cys residues at positions A and B of the V3 region of the major envelope protein gp120 of HIV-1.

In a further embodiment the above subregion is recognized by at least 85% of randomly selected sera of individuals infected with the retrovirus from which it is derived.

Preferred embodiments of the present invention are

a) DNA sequences encoding a subregion mentioned above,

b) recombinant vectors containing above DNA sequences,

c) recombinant viruses containing above DNA sequences,

d) a host transformed with a recombinant vector containing above DNA sequences or above recombinant viruses.

Another preferred embodiment of the invention is any method for the production of the above subregion and for cultivating a transformed host in a suitable medium and recovering said subregion from the culture.

Another preferred embodiment of the invention is the the above subregion which is chemically synthesized.

The invention also relates to compositions containing the above subregion. Preferably said compositions are useful for the diagnosis, prophylaxis or therapy of retrovirus infections, preferably of HIV-1 infections.

The most dominant HIV-1 neutralizing epitope resides within the third variable domain V3 of the outer structural membrane protein gp120. This epitope was first described by our group as the only antigenic region without consensus sequence for N-glycosylation and discussed in its significance for immune response (Modrow et al., 1987). This importance of V3 as a major neutralizing epitope was experimentally confirmed by several groups ( Meloen et al.).

As a consequence of the importance and typespecifity of neutralizing antibodies, we designed a 36 amino acid consensus peptide including the highly conserved GPGR-amino acid motiv, a T-cell epitope and two highly conserved cystein residues being possibly important for secondary and tertiary structure of the peptide. The design of the V3 consensus peptide is based on the comparison of 20 different and independent HIV-1 isolates derived from Europe, USA and Africa (Fig. 1). The V3 consensus peptide was chemically synthesised and purified as described in the examples using a peptide synthesizer (Milligen 9050).

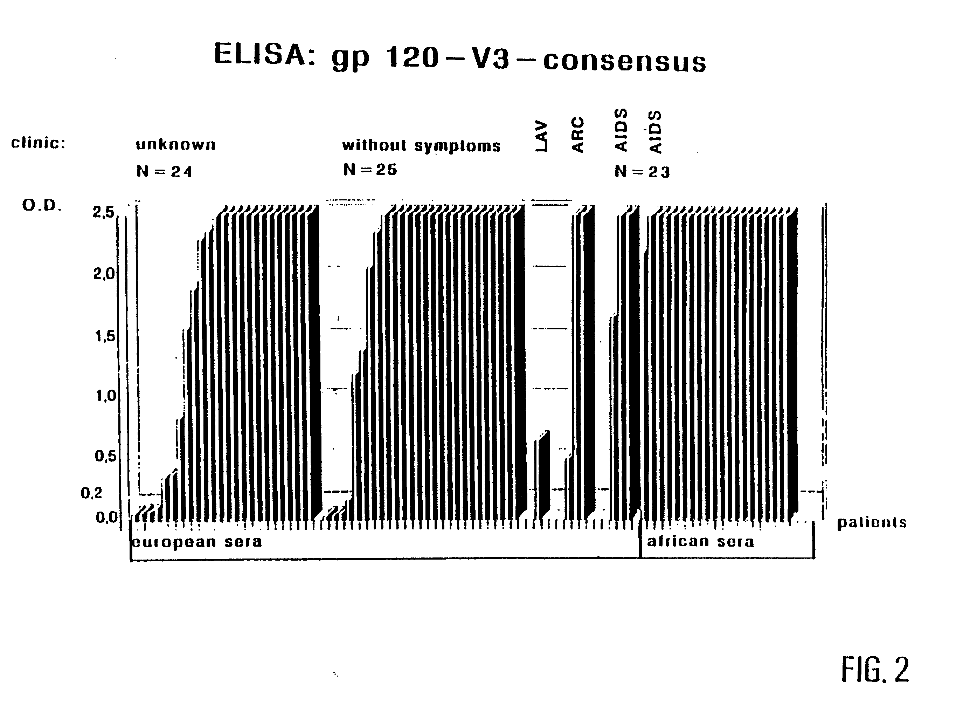

In contrast to publications concerning seroactivities of V3-sequences derived directly from defined isolates, almost 90% of randomly selected sera of HIV-1 infected persons reacted with the designed V3 consensus peptide. At least 85% of investigated sera from HIV-1 patients without symptoms recognized the V3 consensus peptide in ELISA. All of the LAV-, ARC- and AIDS-sera tested were positiv in ELISA independently of their origin (Europe and Africa; fig. 2).

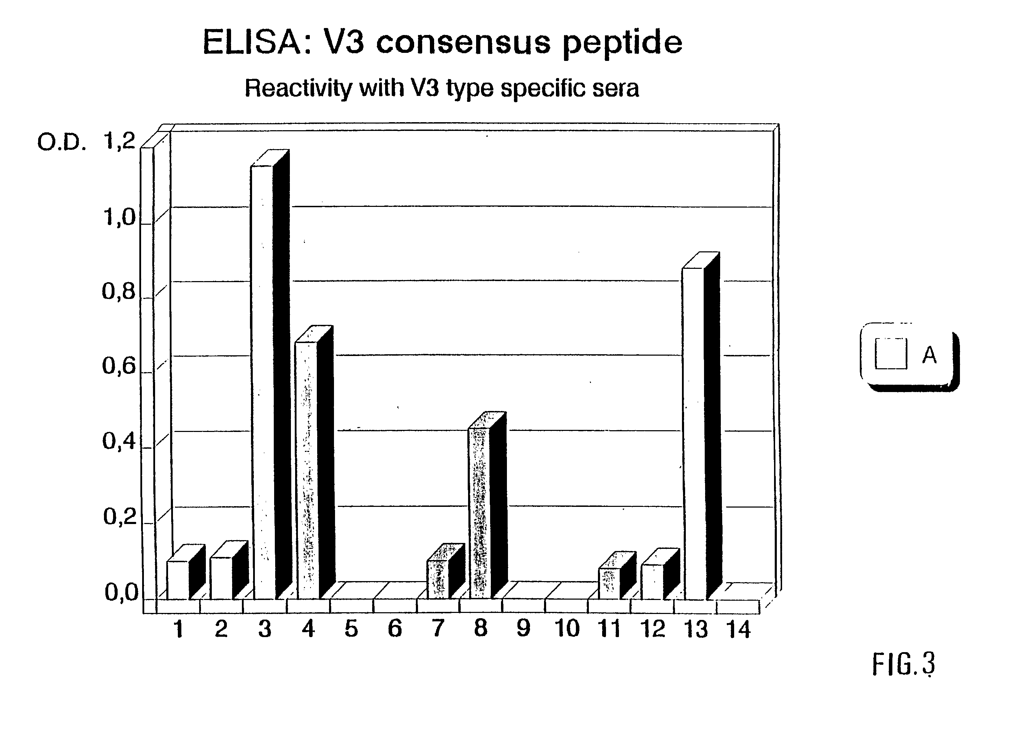

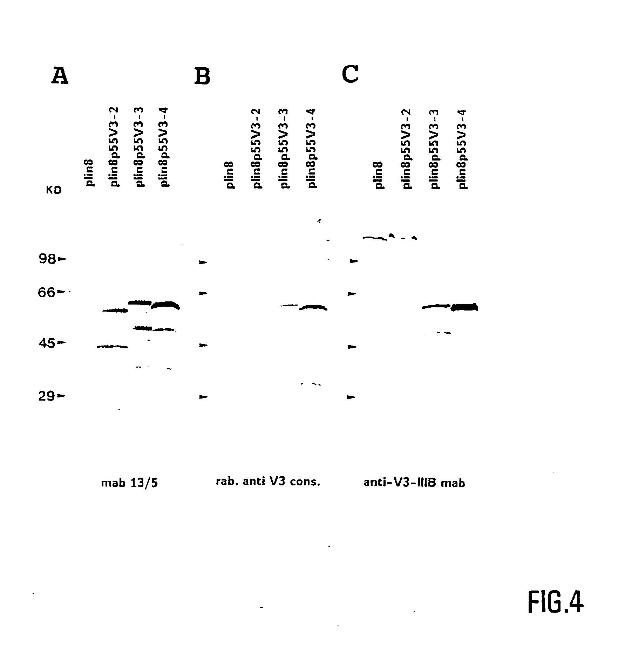

Another approach analysed the reactivity of typespecific sera raised against defined short peptides (Palker et al., 1988) representing the V3 neutralizing epitope of various defined virus families with the V3 consensus peptide: HTLVIIIb-V3 monoclonal antibodies as well as IIIb-V3 and MN-V3 specific peptide sera recognized the V3 consensus peptide in ELISA. An RF-strain specific anti V3-serum reacted weakly with the V3 consensus peptide. Another anti peptide serum, raised against a short V3-consensus sequence (12aa) developed in our laboratory (Modrow et al. (1989)) reacted positively in ELISA-tests (fig. 3). Moreover using the V3 consensus peptide as an immunogen we were able to induce polyclonal anti-V3-antibodies in rabbits as demonstrated by Western blot analysis (fig. 4).

As a consequence of all findings we conclude that a V3 consensus sequence was designed, which is recognized by a wide spectrum of different sera. Broad reactivity is, in addition to the presence of a consensus motiv, possibly due to the fact that more than one B-cell epitope is located on the 36 amino acid V3 consensus peptide. Moreover, the conserved flanking cysteins possibly enable at least part of the peptide to form the original 3D configuration and cause thereby the recognition of conformational epitopes by the sera tested. The V3 consensus corresponding DNA sequence will be an important part for recombinant subunit vaccines.

Brief description of the figures

| | |
|---|---|
| Fig. 1: | gp120-V3 consensus sequence based on a comparison of the depicted V3 regions derived from different independent virus strains. Bold underlined capitals depict highly conserved amino acids. Small underlined letters show amino acids being less |

conserved and small letters not underlined represent highly variable amino acids.

| | |
|---|---|
| Fig. 2: | Evaluation of the V3 consensus sequence by a peptide ELISA using patient sera of different clinical stages. To exclude type specific or geographically correlatable reaction sera of different continents (Europe and Africa) were used. |

*Cut of* was selected 10 times as high as the standart negative control (Behring Werke AG, Marburg, FRG).

| | |
|---|---|
| Fig. 3: | Elisa, testing the reaction of the V3 consensus peptide coupled to the solid phase with sera or monoclonal antibodies raised against short peptides representing the gp120/V3 domain (aa303-321) of different defined HIV-1 isolates. |
| lane 1: | Goats were immunized with tetanus toxoid MBS. The resultant serum serves as a negative control to antisera raised against the V3 domain of gp120RF, IIIB and MN (lane 2-4). |
| lane 2-4: | synthetic peptides, containing a type specific determinant of the third variable domain V3 of gp120 were coupled to tetanus toxoid and used to raise goat antibodies against HIV gp120. |
| lane 2: | reactivity of serum raised against the V3 domain of gp120RF |
| lane 3: | reactivity of serum raised against the V3 domain of gp120IIIB |
| lane 4: | reactivity of serum raised against the V3 domain of gp120MN |
| lane 7: | rabbit preimmune serum, serves as a negative control for an antiserum raised against a short V3/gp120 consensus peptide. |
| lane 8: | rabbit serum, raised against a short V3/gp120 consensus sequence coupled to KLH. |
| lane 11-12: | anti p24 and anti RT mouse monoclonal antibodies serving as a negative control for a mouse monoclonal antibody directed towards the gp120IIIB V3 domain. |
| lane 13: | anti gp120IIIB-V3 mouse monoclonal antibody |
| Fig. 4: | Bacterial expression of chimeric p55/V3 proteins, which contained the gp120/V3 consensus domain |

integrated into different positions of the 55 core protein (plin8p55V3-2,-3,-4). Using conventional Western blot analysis, the recombinant p55V3 fusion proteins were specifically detected by monoclonal antibodies directed to p24 (a) and (b) to the V3 epitope of a foreign isolate (IIIb). A polyclonal serum raised against a synthetic peptide, representing the original sequence of the V3 consensus sequence, detected the p55/V3 fusion proteins even better than the IIIb-peptide derived monoclonal antibody. The fact that neither the monoclonal antibody, nor the polyclonal V3 specific serum detect the p55V3-1 protein indicates, that the antigenic context of the V3 region is important for immunological detection.

**Theexamples illustrate the invention** (standart methods: Sambrook et al., 1989)

Example 1: Design of the consensus sequence

The design of the V3 consensus sequence is based on the comparison of 20 different and independent HIV-1 isolates derived from Europe, USA and Africa. The derived sequence encloses the highly conserved GPGR motiv, a T-cell epitope, an ADCC epitope and the flanking cystein residues in order to enable the peptide to form a disulfide bridge. (Fig. 1)

Example 2: Peptide synthesis

Peptide synthesis was done in a 9050 peptide synthesizer (Milligen) using Fmoc (9-fluorenylmethyloxycarbonyl)-protected amino acids. The first residue was coupled to the resin by an acid labile amide anchor group (Tentagel AM, purchased by Rapp polymere, Tübingen FRG). t-Butyl (Ser, Thr, Tyr, Cys, Asp, Glu, His), t-butoxycarbonyl (Lys), and 4-methoxy-2,3,6-trimethylbencenesulfonyl (Arg) groups were used for side chain protection. Fmoc protected amino acids were converted to hydroxybenzotriazol-activated esters by treatment with 0,75 mmol of hydroxybenzotriazol and 0,6 mmol diisopropylcarbodiimide per mmol of amino acid for 10 min in dimethylformamide. The subsequent coupling reaction was performed in dimethylformamide. After the coupling reactions, Fmoc groups were removed with 20% piperidine in dimethylformamide followed by a series of washes with dimethylformamide (all solvents were purchased from Merck AG, Darmstadt, F.R.G.; Fmoc protected aminoacids were from Bachem AG, Bubendorf, Switzerland; all chemicals from Aldrich, Steinheim, F.R.G.). After synthesis, side chain protecting groups were removed by a 12 h treatment in trifluoracetic acid containing 5% mercaptoethanol, 3% thioanisol, 3% phenol at room temperature. The peptide was precipitated in an excess of ice cold t-butyl-ethyl ether, washed several times and suspended in 1,5% ammoniumbicarbonate. The peptide was lyophilized and purified by reversed phase high performance liquid chromatography (HPLC) using a gradient of 0-70% acetonitrile in 0,1% trifluoracetic acid. The sequences of the peptide were checked by amino acid analysis and the peptides were stored as lyophilized material at 4°C.

Example 3: ELISA Test

Eight hundred nanograms of uncoupled purified peptide per well were coupled over night in 0,2 M sodium carbonate buffer, pH 9,5 to 96 well microtiter plates (Dynotech). Free protein binding sites were saturated by a two hours incubation with gelatin solution (5 mg/ml, Sigma Chemicals, Munich, F.R.G.). Before and after addition of the respective serum dilutions (2h, 37°C) in PBS ( patient sera:1/100, antipeptide specific goat and rabbit sera 1/16, monoclonal antibodies 1/800 in phosphate buffered saline), the plates were washed several times with PBS 0.5% Tween 20. The second antibodies were added in a dilution of 1/1000 in PBS/0,5% Tween 20. Staining was done in 0,1 M phosphate buffer, pH 6,0 containing 0,5 mg/ml o-phenylenediamin and 0,1% $H_2O_2$ for 10 min, the reaction was stopped with 1 M $H_2SO_4$, and optical density was determined at 486 nm. (Figs 2/3)

Example 4: Immunization of rabbits

For the initial use as immunogen in rabbits, 200 $\mu$g of the dissolved V3 consensus peptide were used in an emulsion with complete Freund's adjuvant. Boostering was performed four times with 200 $\mu$g of the V3 consensus peptide in an emulsion of incomplete Freund's adjuvant in four week intervals.

Example 5: Evaluation of raised anti-V3 consensus peptide antiserum by Western blot analysis

The V3 consensus peptide derived pepide serum was tested by ELISA and by conventional Western blot analysis. For this reason, the V3 consensus sequence was integrated into three different sites of bacterial p55 expression clones. These clones were induced with 2mM IPTG at an O.D. of 0,6. The E. coli pellet was resuspended after 2 h in boiling mix. The resulting recombint p55/V3 fusion proteins were detected after conventional Western blot with the anti-V3 consensus peptide derived rabbit serum. (Fig. 4)

**References:**
Blattner W., Nara P., Shaw G., et al.,1989, Prospective clinical, immunological and virologoc follow up of an infected lab worker,, V international Conference on AIDS, Montreal, (abs. M.C.O.7)
Goudsmid, J., Linear versus conformational V3 heterogeneity, (1989), AIDS, Vol 3
Goudsmit J., De Jong J., Epstein L., et al., Genomic and antigenic variation in the gp120 neutralization domain V3: challenge to an AIDS vaccine, 1990,UCLA symposia on molecular and cellular Biology, in press
Goudsmit J., Zwart G., Bakker M., Epstein L., Kuiken C., De Wolf F., 1989a, Natural antibody perception of amino acid divergence within an HIV-1 nutralizing epitope, 1989, V int. conf. on AINS, Montreal, (abs. W.C.P.44)
Goudsmit, I., Debouck, C., Moloen, R.H., Smit, L., Bakker, M., Asher, D.M., Wolff, A.V., Gibbs, C.J., Gajdusch, D.C. (1988) Human immundeficiency virus type 1 neutralization epitope with conserved architecture elicts early type specific antibodies in experimentally infected chimpanzees. PNAS-USA, Vol. 85, p. 4478.
Hattori, T., Koito, A., Takatsuki, K., Kido, H., Katunuma, N. (1989) Involvement of tryptase-related cellular proteases in human immunodeficiency virus type 1 infection. FEBS-Lett., Vol. 248, p. 48-52.
Javaherian K., Langlois, J. A., McDanal, C., Ross, K. L., Eckler, L. J., Jellis, C. L., Profy, A., T., Rusche, J. R., Bolognesi, D. P., Putney, S. D. et al., 1989, Principal neutralizing domain of the human immunodeficiency virus type 1 envelope protein, PNAS-USA, pp6768
Kong L.I., Taylor M.E., Waters D., Blattner W. A., Hahn B. H., Shaw G. M., 1989, Genetic analysis of sequential HIV-1 isolates from an infected lab worker, V int. Conf. on AIDS, Montreal, (abs. T.C.O.19)
Lyerly H. K., Matthews T. J., Langlois, A. J., Bolognesi, D. P., Weinhold, K. J., 1987, Human T-cell lymphotropic virus IIIB glycoprotein bound to CD4 determinants on normal lymphocytes and expressed by infected cells serves as a target for immune attack, PNAS-USA, Vol. 84, pp 4601
Lyerly H. K., Reed D. L., Matthews T. J., 1988, Anti gp120 antibodies from HIV seropositive individuals mediate broad reactive anti-HIV ADCC, AIDS Res Hum Retrovirus, Vol. 3, pp 409
Modrow, S., Hahn, B. H., Shaw, G. M., Gallo, R. C., Wong-Stahl, F., Wolf, H., 1987. Computer-assisted Analysis of Envelope Protein Sequences of seven Human Immunodeficiency Virus Isolates: Prediction of Antigenic Epitopes in Conserved and Variable Regions. J Virol 61, 570-578.
Modrow, S., Höflacher, B., Mellert, W., Erfle, V., Wahren, B., Wolf, H., (1989). Use of Synthetic Oligopeptides in Identfication and Characterization of Immunological Functions in the Amino Acid Sequence of the Envelope Protein of HIV-1. J ac Imm Def Syn 2, 21-27.
Myers, G., Josephs, S. F., Rabson, A. B., Smith, T. F., Wong-Stahl, F., 1988. Human Retroviruses and AIDS. Los Alamos National Laboratory, Los Alamos, N.M.
Palker, T.J., Clark, M.E., Langlois, A.J., Matthews, T.J., Weinhold, K.J., Randall, R.R., Bolognesi, D.P., Haynes, B.F. (1988) Type specific neutralization of the human immunodeficiency virus with antibodies to env-encoded synthetic peptides. PNAS-USA, Vol. 85, p. 1932.
Puttney S.D., LaRosa G., Javaherian K., Emini E., Bolognesi D., Matthews T., 1989, Development of an AIDS vaccine; V int. conf. on AIDS, Montreal, (abs. W.C.O.18)
Skinner M. A., Langlois, A. J., McDanal, C. B., Mc Dougal, J. S., Bolognesi, D. P., Mathews, T.,J., 1988, Neutralizing antibodies to immunodominant envelope sequenz do not prevent gp120 binding to CD4, J: Virol., Vol. 62, pp4195
Takahashi, H., Cohen, J., Hosmalin, A., Cease K.B., Houghten, R., Cornette, I.L., De Lisi, C., Moss, B., Germain, R.N., Berzofsky, I.A. (1988) An immunodominant epitope of the human immunodeficiency virus envelope glycoprotein gp120 recognized by class I major histocompatibility complex molecule-restricted murine cytotoxic T-lymphocytes. PNAS-USA, Vol. 85, p. 3105.

**Claims**

1. A subregion of the retroviral env protein displaying the following features:

   a) it is biologically equivalent to the V3 region of the HIV-1 env protein;

   b) it is recognized by at least 70% of randomly selected sera of individuals infected with the retrovirus from which it is derived;

   c) it contains a strictly conserved oligopeptide;

   d) it contains a helper T-cell epitope;

   e) it contains at least two Cys residues which are essential for the formation of its secondary and /or tertiary structure; and

   f) its sequence is designed by using the V3 region of a given HIV-1 env protein and introducing deletions observed in the majority of other HIV-1 isolates, or by comparing the V3 regions of given HIV-1 env proteins and determining a consensus sequence which optionally includes deletions observed in the majority of HIV-1 isolates.

2. The subregion according to claim 1 wherein an additional deletion is inserted which corresponds to position 36 of figure 1.

3. The subregion according to claim 1 or 2 which is the V3 region of the major envelope protein gp120 of HIV-1.

4. The subregion according to claim 3 which essentially consists of an amino acid sequence corresponding to the amino acid sequence between positions aa294 and aa332 of said gp120 of HIV-1 isolate.

5. The subregion according to any one of claims 1 to 4, wherein the strictly conserved oligonucleotide has a length of about 3 to 10 amino acids.

6. The subregion according to any one of claims 1 to 5, wherein the strictly conserved oligonucleotide has the sequence GPGR.

7. The subregion according to any one of claims 1 to 6 which contains the two Cys residues at positions A and B of the V3 region of the major envelope protein gp120 of HIV-1.

8. The subregion according to any one of claims 1 to 7 which is recognized by at least 80% of randomly selected sera of individuals infected with the retrovirus from which it is derived.

9. A DNA sequence encoding a subregion according to any one of claims 1 to 8.

10. A recombinant vector containing a DNA sequence according to claim 9.

11. A recombinant virus containing a DNA sequence according to claim 9.

12. A host transformed with a recombinant Vector according to claim 10 or a recombinant virus according to claim 11.

13. A method for the production of a subregion according to any one of claims 1 to 8 Comprising the steps of cultivating a transformed host according to claim 12 in a suitable medium and recovering said subregion from the culture.

14. A subregion according to any one of claims 1 to 8 which is chemically synthesized.

15. A composition containing a subregion according to any one of claims 1 to 8

16. The composition according to claim 15 for the diagnosis, prophylaxis or therapy of retrovirus infections.

17. The composition according to claim 16 wherein the retrovirus infection is an HIV-1 infection.

**Claims for the following Contracting State: GR**

1. A subregion of the retroviral env protein displaying the following features:
   a) it is biologically equivalent to the V3 region of the HIV-1 env protein;
   b) it is recognized by at least 70% of randomly selected sera of individuals infected with the retrovirus from which it is derived;
   c) it contains a strictly conserved oligopeptide;
   d) it contains a helper T-cell epitope;
   e) it contains at least two Cys residues which are essential for the formation of its secondary and /or tertiary structure; and
   f) its sequence is designed by using the V3 region of a given HIV-1 env protein and introducing deletions observed in the majority of other HIV-1 isolates, or by comparing the V3 regions of given HIV-1 env proteins and determining a consensus sequence which optionally includes deletions observed in the majority of HIV-1 isolates.

2. The subregion according to claim 1 wherein an additional deletion is inserted which corresponds to position 36 of figure 1.

3. The subregion according to claim 1 or 2 which is the V3 region of the major envelope protein gp120 of HIV-1.

4. The subregion according to claim 3 which essentially consists of an amino acid sequence corresponding to the amino acid sequence between positions aa294 and aa332 of said gp120 of HIV-1 isolate.

5. The subregion according to any one of claims 1 to 4, wherein the strictly conserved oligonucleotide has a length of about 3 to 10 amino acids.

6. The subregion according to any one of claims 1 to 5, wherein the strictly conserved oligonucleotide has the sequence GPGR.

7. The subregion according to any one of claims 1 to 6 which contains the two Cys residues at positions A and B of the V3 region of the major envelope protein gp120 of HIV-1.

8. The subregion according to any one of claims 1 to 7 which is recognized by at least 80% of randomly selected sera of individuals infected with the retrovirus from which it is derived.

9. A DNA sequence encoding a subregion according to any one of claims 1 to 8.

10. A recombinant vector containing a DNA sequence according to claim 9.

11. A recombinant virus containing a DNA sequence according to claim 9.

12. A host transformed with a recombinant Vector according to claim 10 or a recombinant virus according to claim 11.

13. A method for the production of a subregion according to any one of claims 1 to 8 comprising the steps of cultivating a transformed host according to claim 12 in a suitable medium and recovering said subregion from the culture.

14. A subregion according to any one of claims 1 to 8 which is chemically synthesized.

15. A composition containing a subregion according to any one of claims 1 to 8.

16. Use of a subregion according to any of claims 1 to 8 for the preparation of a pharmaceutical composition.

17. Use according to claim 16 wherein the composition is useful for the diagnosis, prophylaxis or therapy of retrovirus infections.

18. Use according to claim 17 wherein the retrovirus infection is an HIV-1 infection.

**Claims for the following Contracting State: ES**

1. A method for the production of a subregion of the retroviral env protein displaying the following features:
   a) it is biologically equivalent to the V3 region of the HIV-1 env protein;
   b) it is recognized by at least 70% of randomly selected sera of individuals infected with the retrovirus from which it is derived;
   c) it contains a strictly conserved oligopeptide;
   d) it contains a helper T-cell epitope;
   e) it contains at least two Cys residues which are essential for the formation of its secondary and/or tertiary structure; and
   f) said method comprising designing the sequence of said subregion by using the V3 region of a given HIV-1 env protein and introducing deletions observed in the majority of other HIV-1 isolates, or by comparing the V3 regions of given HIV-1 env proteins and determining a consensus sequence which optionally includes deletions observed in the majority of HIV-1 isolates.

2. The method according to claim 1 wherein an additional deletion is inserted which corresponds to position 36 of figure 1.

3. The method according to claim 1 or 2 wherein the subregion is the V3 region of the major envelope protein gp120 of HIV-1.

4. The method according to claim 3 wherein the subregion essentially consists of an amino acid sequence corresponding to the amino acid sequence between positions aa294 and aa332 of said gp120 of HIV-1 isolate.

5. The method according to any one of claims 1 to 4, wherein the strictly conserved oligonucleotide has a length of about 3 to 10 amino acids.

6. The method according to any one of claims 1 to 5, wherein the strictly conserved oligonucleotide has the sequence GPGR.

7. The method according to any one of claims 1 to 6 wherein the subregion contains the two Cys residues at positions A and B of the V3 region of the major envelope protein gp120 of HIV-1.

8. The method according to any one of claims 1 to 7 wherein the subregion is recognized by at least 80% of randomly selected sera of individuals infected with the retrovirus from which it is derived.

9. The method according to any one of claims 1 to 8 wherein the subregion is chemically synthesized.

10. A method for the production of a DNA sequence encoding a subregion obtainable by a method of any one of claims 1 to 9, said method comprising the chemical synthesis of said DNA sequence, its isolation from a natural source and/or its construction by recombinant DNA techniques.

11. A method for the production of a recombinant vector, said method comprising the insertion of a DNA sequence obtainable by the method of claim 10 into a vector molecule.

12. A method for the production of a recombinant virus, said method comprising the insertion of a DNA sequence obtainable by the method of claim 10 into a viral DNA.

13. A method for the production of a host containing a recombinant vector obtainable by a method according to claim 11 or a recombinant virus obtainable by a method according to claim 12, said method comprising the incorporation of said recombinant vector or said recombinant virus into a suitable host.

14. A method for the production of a subregion as defined in any one of claims 1 to 9, said method comprising the steps of cultivating a transformed host obtainable by a method according to claim 13 in a suitable medium and recovering said subregion from the culture.

```
HXB2    NCTRPNNN..TRKR.IRIQRGPGRAFVTIGK....IGNMRQAHCN
BRU     ---------..---S.------------------....----------
NL43    ---------..---S.------------------....----------
SF2     ---------..---S.-Y-..------H-T-R...I--DI-K----
MN      ------Y-K..---.-H-..------Y-TKN...I--TI------
SC      ---------  -TRS.-H-..------YAT-D...I--DI------
JH3     -----S...K-TR-R-H-..------Y-TKQ...IA-DL------
BRVA    ---------..----.-TM..----VYY-T-Q...I--DI-R----
CDC4    -------H..----.VTL..----VWY-T-E...IL--I------
NY5     ---------..-K-G.-A-..----TLYARE-...I--DI------
WMJ2    -----Y--..V-RS.LS-..------R-RE....I--II------
RF      ---------..---S.-TK..----VIYAT-Q...I--DI-K----

MAL     -----G--..--RG.-HF..---Q-LY-T-....IV-DI-R-Y-T
ELI     T-A--YQ-:..--Q-.TP-..-L-QSLY-TRSR.SI--...-----
Z6      -----YK-..--QS.TP-..-L-Q-LY-TRGRTKI--...-----
Z2      -----YR-..I-Q-.TS-..-L-Q-LY-TKTR.SI--...--Y--
Z3      -----GSDKKI-QS.---..---KV-YAK-G...IT-...-----

V3-CONS.  qCtRPnnn..tRkr iri..GpGrafvttgk...I.gnmrqAhCt
```

FIG.1

# ELISA: gp 120 – V3 – consensus

FIG. 2

EP 0 448 095 A1

FIG.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,P | WO-A-9 003 984   (REPLIGEN CO.)(19-04-1990)<br>* Table I on page 49; example 3 *<br>— — — | 1-17 | A 61 K 39/21<br>C 12 N 15/49<br>C 12 N 5/10 |
| X | PROC. NATL. ACAD. SCI. USA, vol. 85, May 1988, pages 3198-3202; J.R. RUSCHE et al.: "Antibodies that inhibit fusion of human immunodeficiency virus-infected cells bind a 24-amino acid sequence of the viral envelope, 9p 120"<br>* Table 5 *<br>— — — | 1-8,14-17 | |
| X,D | PROC. NATL. ACAD. SCI. USA, vol. 86, no. 17, September 1989, pages 6768-6772; K. JAVAHERIAN et al.: "Principal neutralizing domain of the human immunodeficiency virus type 1 envelope protein"<br>* Figure 1 *<br>— — — | 1-8,14-17 | |
| A | EP-A-0 311 219   (STICHTING CENTRAAL DIER-GENEESKUNDIG INSTITUUT)(12-04-1989)<br>* Column 3, lines 44-53 *<br>— — — | 1-8,14-17 | |
| A | EP-A-0 306 219   (REPLIGEN CORP.)(08-03-1989)<br>* The whole document *<br>— — — | 1-17 | |
| A | WO-A-8 800 471   (SOUTHWEST FOUNDA-TION)(28-01-1988)<br>* Page 27, table II, residue no. 2 *<br>— — — — — | 1-8,14-17 | |

TECHNICAL FIELDS
SEARCHED (Int. Cl.5)

C 07 K
C 12 N
C 12 P
A 61 K

The present search report has been drawn up for all claims

| Place of search | Date of completion of search | Examiner |
|---|---|---|
| The Hague | 27 June 91 | CUPIDO M. |